(19) Europäisches Patentamt
European Patent Office
Office européen des brevets

(11) Veröffentlichungsnummer: **0 033 456**
**B1**

(12) # EUROPÄISCHE PATENTSCHRIFT

(45) Veröffentlichungstag der Patentschrift: **22.12.82**

(51) Int. Cl.³: **C 07 F 9/65,** A 01 N 57/16, A 01 N 57/32 // C07D285/00

(21) Anmeldenummer: **81100320.1**

(22) Anmeldetag: **17.01.81**

(54) **Heterocyclische Phosphorsäurederivate, Verfahren zu ihrer Herstellung und ihre Verwendung zur Bekämpfung von Schädlingen.**

(30) Priorität: **04.02.80 DE 3003977**

(43) Veröffentlichungstag der Anmeldung:
**12.08.81 Patentblatt 81/32**

(45) Bekanntmachung des Hinweises auf die Patenterteilung:
**22.12.82 Patentblatt 82/51**

(84) Benannte Vertragsstaaten:
**BE CH DE FR GB IT LI NL**

(56) Entgegenhaltungen:
**DE-A-2 336 827**
**DE-A-2 527 676**
**US-A-4 139 363**

(73) Patentinhaber: **BASF Aktiengesellschaft, Carl-Bosch-Strasse 38, D-6700 Ludwigshafen (DE)**

(72) Erfinder: **Baumann, Annegrit, Dr., Im Sennteich 26, D-6800 Mannheim (DE)**
Erfinder: **Kiehs, Karl, Dr., Sudetenstrasse 22, D-6840 Lampertheim (DE)**
Erfinder: **Hamprecht, Gerhard, Dr., Rote-Turm-Strasse 28, D-6940 Weinheim (DE)**
Erfinder: **Lange, Arno, Dr., Friedrichplatz 11, D-6800 Mannheim (DE)**
Erfinder: **Adolphi, Heinrich, Dr., Kalmitweg 11, D-6703 Limburgerhof (DE)**

**EP 0 033 456 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Heterocyclische Phosphorsäurederivate, Verfahren zu ihrer Herstellung und ihre Verwendung zur Bekämpfung von Schädlingen

Die vorliegende Erfindung betrifft heterocyclische Phosphorsäurederivate, Verfahren zu ihrer Herstellung, Schädlingsbekämpfungsmittel, die diese Verbindungen als Wirkstoffe enthalten, sowie ein Verfahren zur Bekämpfung von Schädlingen mit diesen Verbindungen.

Die erfindungsgemässen Verbindungen haben die Formel I

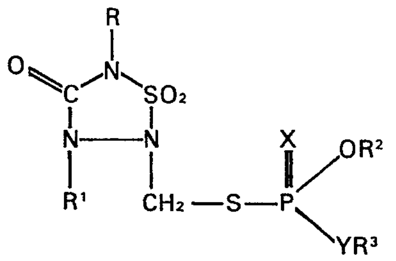

(I),

in der

R gegebenenfalls durch Methoxy oder Halogen substituiertes Alkyl mit bis zu 10 Kohlenstoffatomen oder

Cycloalkyl mit 4 bis 8 Kohlenstoffatomen,

$R^1$ Alkyl mit bis zu 6 Kohlenstoffatomen oder Cycloalkyl mit 4 bis 8 Kohlenstoffatomen,

$R^2$ Alkyl mit bis zu 5 Kohlenstoffatomen,

$R^3$ Alkyl mit bis zu 5 Kohlenstoffatomen,

X Sauerstoff oder Schwefel und

Y Sauerstoff, Schwefel oder den bivalenten Rest $-NR^4-$, wobei $R^4$ für Wasserstoff oder Alkyl mit bis zu 5 Kohlenstoffatomen steht, bedeuten.

Die Verbindungen eignen sich zur Bekämpfung von Schädlingen, insbesondere von Insekten, Spinnentieren und Nematoden. Sie sind bekannten Wirkstoffen aus der Klasse der Phosphorsäureester, wie O,O-Diethyl-S-(2-chlor-1-phthalimidoethyl)-phosphordithioat (US-PS 2984669) und O,O-Dimethyl-S-(N-methylcarbamoyl-methyl)-phosphordithioat (DE-PS 819998), in ihrer Wirkung überlegen. Die erfindungsgemässen Verbindungen können im Pflanzenschutz sowie auf dem Hygiene-, Vorratsschutz- und Veterinärsektor als Schädlingsbekämpfungsmittel eingesetzt werden.

R in Formel I steht für unverzweigte oder verzweigte Alkylreste mit bis zu 10 Kohlenstoffatomen, vorzugsweise bis zu 4 Kohlenstoffatomen, die durch Methoxy oder Halogen, wie Chlor, substituiert sein können, oder für Cycloalkylreste mit 4 bis 8 Kohlenstoffatomen. Beispiele für derartige Reste sind Methyl, Ethyl, Isopropyl, 2-Chlorethyl, n-Butyl, sec.-Butyl, i-Butyl, Cyclobutyl, Cyclopentyl, Cyclohexyl, Cycloheptyl, Cyclooctyl.

$R^1$ in Formel I steht für unverzweigte oder verzweigte Alkylreste mit bis zu 4 Kohlenstoffatomen, wie Methyl, Ethyl, n-Propyl, i-Propyl, sec.-Butyl, i-Butyl, oder Cycloalkylreste mit 4 bis 8 Kohlenstoffatomen, wie Cyclobutyl, Cyclohexyl, Cyclooctyl. $R^3$ bedeutet unverzweigte oder verzweigte Alkylreste mit bis zu 5 Kohlenstoffatomen, wie Methyl, Ethyl, n-Propyl, i-Propyl, n-Butyl, n-Pentyl, i-Pentyl. Vorzugsweise stehen $R^2$ für Ethyl und $R^3$ für einen Butylrest.

Die heterocyclischen Phosphorsäurederivate der Formel I können durch Umsetzung von 1,2,3,5-Thiatriazolidin-4(2H)-on-1,1-dioxid-derivaten der Formel II

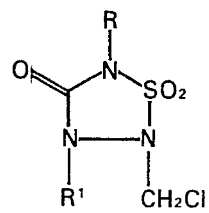

(II),

in der

R und $R^1$ die obengenannten Bedeutungen haben, mit einem Phosphorsäureestersalz der Formel III

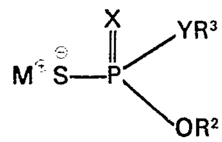

(III),

in der

$R^2$, $R^3$, X und Y die obengenannten Bedeutungen haben und $M^{\oplus}$ für ein gegebenenfalls substituiertes Ammoniumion, ein Alkalimetallion oder ein Äquivalent Erdalkalimetallion steht,

in Gegenwart eines inerten organischen Lösungsmittels erhalten werden.

Die Umsetzung wird im allgemeinen bei einer Temperatur im Bereich von 10 bis 100°C, vorzugsweise 40 bis 60°C, in Abhängigkeit vom Siedepunkt des Lösungsmittels, drucklos oder unter Druck, diskontinuierlich oder kontinuierlich durchgeführt.

Geeignete Lösungsmittel sind beispielsweise aliphatische und aromatische, gegebenenfalls chlorierte oder nitrierte Kohlenwasserstoffe, wie Benzol, Toluol, Xylol, Chlorbenzol, Chloroform, Methylenchlorid, Tetrachlorkohlenstoff; cyclische und acyclische Ether, wie Diethylether, Tetrahydrofuran; Ketone, wie Aceton, Cyclohexanon; Nitrile, wie Acetonitril. Auch Gemische dieser Lösungsmittel können verwendet werden.

Zweckmässigerweise setzt man die Ausgangsstoffe der Formeln II und III in äquimolarem Verhältnis ein. Ein Überschuss der einen oder anderen Reaktionskomponente kann in einigen Fällen Vorteile bringen.

Die als Ausgangsverbindungen verwendeten 1,2,3,5-Thiatriazolidin-4(2H)-on-1,1-dioxidderivate der Formel II können durch Umsetzung von Sulfamidsäurechloriden der Formel IV

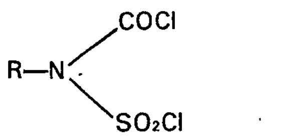

(IV)

mit Hydrazinen der Formel V

$H_2N$-NH-$R^1$ (V)

zu 1,2,3,5-Thiatriazolidin-4(2H)-on-1,1-dioxiden der Formel VI

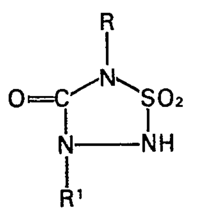

(VI),

die durch Umsetzung mit Formaldehyd und Thionylchlorid in die entsprechenden Chlormethylsubstituierten Verbindungen überführt werden können, erhalten werden. Die Substituenten R und $R^1$ in diesen Formeln haben dabei die obengenannten Bedeutungen (J. Chem. Research 1977, S. 2813 bis 2825).

Die Phosphorsäureestersalze der Formel III sind bekannt (Houben-Weyl, Methoden der organ. Chemie, Bd. 12/2, S. 690 ff, Georg Thieme-Verlag, Stuttgart, 1964).

Als Kationen dieser Salze kommen ausser dem Ammoniumion substituierte Ammoniumionen, beispielsweise das Dimethylammoniumion, das Diäthylammoniumion, oder Alkalimetallionen, beispielsweise das Natriumion oder das Kaliumion, oder Erdalkalimetallionen, beispielsweise das Calciumion, in Betracht.

Die folgenden Beispiele erläutern die Herstellung der erfindungsgemässen Verbindungen.

Beispiel 1

Zu 38, 6 g 3-Methyl-5-isopropyl-1,2,3,5-thiatriazolidin-4(2H)-on-1,1-dioxid werden 22 g Formaldehyd (als 40%ige Formalinlösung) zugegeben. Das Reaktionsgemisch wird einen Tag lang unter Rückfluss erhitzt, die sich anschliessend abscheidende feste Substanz wird mit Petrolether verrieben, abgesaugt und getrocknet. Man erhält 38,1 g 2-Hydroxymethyl-3-methyl-5-isopropyl-1,2,3,5-thiatriazolidin-4(2H)-on-1,1-dioxid.

NMR-Daten:
Dublett bei 1,5 ppm (6 Protonen)
Singulett bei 3,25 ppm (3 Protonen)
Quartett bei 4,25 ppm (1 Proton)
Singulett bei 4,85 ppm (2 Protonen)
OH-Gruppe bei 6–6,2 ppm.

Zu 37,5 g 2-Hydroxymethyl-3-methyl-5-isopropyl-1,2,3,5-thiatriazolidin-4(2H)-on-1,1-dioxid in 120 ml Chloroform werden 29,8 g Thionylchlorid zugetropft. Man lässt das Reaktionsgemisch anschliessend 5 Stunden unter Rückfluss sieden und über Nacht bei Raumtemperatur stehen. Nach dem Filtrieren erhält man 36 g 2-Chlormethyl-3-methyl-5-isopropyl-thiatriazolidin-4(2H)-on-1,1-dioxid vom Fp. 58 bis 64°C.

12,1 g 2-Chlormethyl-3-methyl-5-isopropyl-thiatriazolidin-4(2H)-on-1,1-dioxid in 120 ml Acetonitril werden bei Raumtemperatur mit 13,6 g O-Ethyl-S-sec.-butyl-dithiophosphorsaurem Dimethylammoniumsalz, gelöst in 60 ml Acetonitril, versetzt.

Die Temperatur erhöht sich um ca. 2°C. Es wird 4 Stunden bei 40°C gerührt, dann über Nacht bei Raumtemperatur. Nach dem Einengen wird in Ether aufgenommen und dreimal mit 100 bis 150 ml Wasser extrahiert. Die getrocknete und filtrierte Etherphase wird eingeengt. Man erhält 14,2 g der Verbindung der Formel

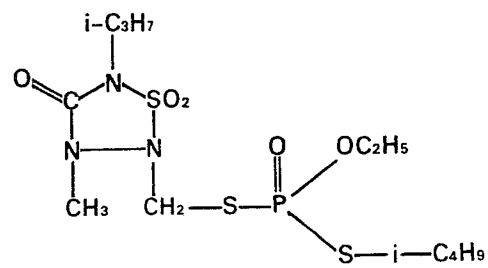

Das Produkt wird säulenchromatographisch an Kieselgel gereinigt (Laufmittel: n-Hexan mit steigendem Aceton-Anteil bis zu 8%); $n_D^{25}$ = 1,5072

| | C | H | N | S | P |
|---|---|---|---|---|---|
| Ber.: | 34,4 | 6,3 | 10,0 | 22,9 | 7,4 |
| Gef.: | 34,8 | 6,3 | 10,3 | 23,1 | 7,2 |

NMR-Daten:
Dublett bei 4,95 ppm (16 Hz) (2 Protonen)
Multiplett bei 4,2 ppm (2+1 Protonen)
Singulett bei 3,25 ppm (3 Protonen)

Beispiel 2

12,1 g 2-Chlormethyl-3-methyl-5-isopropyl-1,2,3,5-thiatriazolidin-4(2H)-on-1,1-dioxid in 100 ml Tetrahydrofuran werden bei 30°C mit 15,5 g O-Ethyl-S-isobutyl-dithiophosphorsaurem Dimethylammoniumsalz, gelöst in Tetrahydrofuran, versetzt. Das Reaktionsgemisch wird 3 Stunden bei 50°C gerührt und über Nacht bei Raumtemperatur gehalten. Dann wird vom Niederschlag abfiltriert, das Filtrat wird eingeengt und mit Dichlormethan aufgenommen. Nach Extraktion mit Wasser wird die Dichlormethanphase getrocknet und eingeengt. Man erhält 15,8 g der Verbindung der Formel

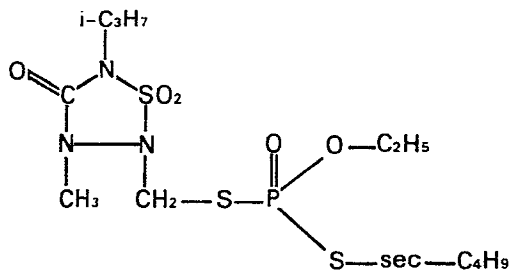

Nach der Reinigung an einer Kieselgelsäule (Laufmittel: n-Hexan/Aceton 8/1) hat das Produkt einen $n_D^{25}$= 1,5121.

| | C | H | N | S | P |
|---|---|---|---|---|---|
| Ber.: | 34,3 | 6,25 | 10 | 22,9 | 7,4 |
| Gef.: | 34,1 | 6,1 | 11,1 | 23,8 | 6,4 |

NMR-Daten:
Dublett bei 4,85 ppm (16 Hz) (2 Protonen)
Multiplett bei 4,15 ppm (2+1 Protonen)
Singulett bei 3,15 ppm (3 Protonen)
Quartett bei 2,7 ppm (6,5 Hz) (2 Protonen)
Multiplett bei 1,9 ppm (1 Proton)
Triplett bei 1,35 ppm (6,5 Hz) (3 Protonen)
Dublett bei 1,45 ppm (6,5 Hz) (6 Protonen)
Dublett bei 1,0 ppm (6,5 Hz) (6 Protonen)

Entsprechend können beispielsweise folgende Verbindungen der Formel I hergestellt werden:

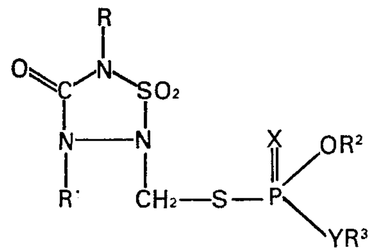

| Nr. | R | R[1] | R² | R³ | X | Y | $n_D^{25}$ |
|---|---|---|---|---|---|---|---|
| 3 | i-$C_3H_7$ | $CH_3$ | n-$C_4H_9$ | $C_2H_5$ | O | S | 1,5138 |
| 4 | i-$C_3H_7$ | $CH_3$ | i-$C_3H_7$ | $C_2H_5$ | O | S | 1,5159 |
| 5 | i-$C_3H_7$ | $CH_3$ | n-$C_3H_7$ | $C_2H_5$ | O | S | 1,5141 |
| 6 | i-$C_3H_7$ | $CH_3$ | $C_2H_5$ | $C_2H_5$ | O | O | 1,5151 |
| 7 | i-$C_3H_7$ | $CH_3$ | $C_2H_5$ | $CH_3$ | O | -NH- | |
| 8 | i-$C_3H_7$ | $CH_3$ | $C_2H_5$ | i-$C_3H_7$ | O | -NH- | 1,4950 |
| 9 | i-$C_3H_7$ | $CH_3$ | $C_2H_5$ | $CH_3$ | O | -N($CH_3$)- | 1,4960 |
| 10 | $C_2H_5$ | $CH_3$ | $C_2H_5$ | n-$C_4H_9$ | O | S | 1,5145 |
| 11 | $C_2H_5$ | $CH_3$ | $C_2H_5$ | i-$C_3H_7$ | O | S | 1,5160 |
| 12 | $C_2H_5$ | $CH_3$ | $C_2H_5$ | $C_2H_5$ | S | O | 1,5170 |
| 13 | $C_2H_5$ | $CH_3$ | $C_2H_5$ | n-$C_3H_7$ | O | S | 1,5150 |
| 14 | $C_2H_5$ | $CH_3$ | $C_2H_5$ | sec-$C_4H_9$ | O | S | 1,5140 |
| 15 | $C_2H_5$ | $CH_3$ | $C_2H_5$ | i-$C_4H_9$ | O | S | 1,5140 |
| 16 | $C_2H_5$ | $CH_3$ | $C_2H_5$ | i-$C_3H_7$ | O | -NH- | 1,4981 |
| 17 | $C_2H_5$ | $CH_3$ | $C_2H_5$ | $CH_3$ | O | -N($CH_3$)- | 1,4980 |
| 18 | $CH_3$ | $CH_3$ | $C_2H_5$ | n-$C_4H_9$ | O | S | 1,5202 |
| 19 | $CH_3$ | $CH_3$ | $C_2H_5$ | sec-$C_4H_9$ | O | S | 1,5195 |
| 20 | $CH_3$ | $CH_3$ | $C_2H_5$ | i-$C_4H_9$ | O | S | 1,5200 |
| 21 | $CH_3$ | $CH_3$ | $C_2H_5$ | i-$C_3H_7$ | O | S | 1,5218 |
| 22 | $CH_3$ | $CH_3$ | $C_2H_5$ | $CH_3$ | O | -N($CH_3$)- | 1,5020 |
| 23 | $CH_3$ | $CH_3$ | $C_2H_5$ | i-$C_3H_7$ | O | -NH- | 1,5019 |
| 24 | $CH_3$ | $CH_3$ | $CH_3$ | i-$C_3H_7$ | O | S | 1,5250 |
| 25 | $CH_3$ | $CH_3$ | $CH_3$ | i-$C_4H_9$ | O | S | |
| 26 | i-$C_3H_7$ | i-$C_3H_7$ | $C_2H_5$ | sec-$C_4H_9$ | O | S | 1,5051 |
| 27 | i-$C_3H_7$ | i-$C_3H_7$ | $C_2H_5$ | i-$C_4H_9$ | O | S | 1,5080 |
| 28 | i-$C_3H_7$ | i-$C_3H_7$ | $C_2H_5$ | i-$C_3H_7$ | O | S | |
| 29 | i-$C_3H_7$ | i-$C_3H_7$ | $CH_3$ | i-$C_4H_9$ | O | S | |
| 30 | $CH_3$ | i-$C_3H_7$ | $C_2H_5$ | sec-$C_4H_9$ | O | S | 1,5155 |
| 31 | $CH_3$ | i-$C_3H_7$ | $C_2H_5$ | i-$C_4H_9$ | O | S | 1,5130 |
| 32 | $CH_3$ | i-$C_3H_7$ | $C_2H_5$ | n-$C_3H_7$ | O | S | Fp: 54-55°C |
| 33 | $CH_3$ | i-$C_3H_7$ | $C_2H_5$ | i-$C_3H_7$ | O | -NH- | 1,4990 |
| 34 | $CH_3$ | i-$C_3H_7$ | $CH_3$ | i-$C_4H_9$ | O | S | |
| 35 | $CH_3$ | i-$C_3H_7$ | $C_2H_5$ | $C_2H_5$ | S | O | 1,5170 |
| 36 | $C_6H_{11}$ | $CH_3$ | $C_2H_5$ | i-$C_4H_9$ | O | S | |
| 37 | $C_6H_{11}$ | i-$C_3H_7$ | $C_2H_5$ | i-$C_4H_9$ | O | S | |
| 38 | $C_6H_{11}$ | $C_6H_{11}$ | $C_2H_5$ | i-$C_4H_9$ | O | S | |
| 39 | i-$C_3H_7$ | $C_6H_{11}$ | $C_2H_5$ | i-$C_4H_9$ | O | S | |

| Nr. | R | $R^1$ | $R^2$ | $R^3$ | X | Y | $n_D^{25}$ |
|---|---|---|---|---|---|---|---|
| 40 | $i-C_3H_7$ | $C_6H_{11}$ | $C_2H_5$ | $n-C_3H_7$ | O | S | |
| 41 | $i-C_3H_7$ | $CH_3$ | $CH_3$ | $i-C_4H_9$ | O | S | |
| 42 | $i-C_3H_7$ | $CH_3$ | $CH_3$ | $n-C_3H_7$ | O | S | 1,5161 |
| 43 | $Cl-CH_2CH_2-$ | $CH_3$ | $C_2H_5$ | $i-C_4H_9$ | O | S | |
| 44 | $ClCH_2CH_2-$ | $CH_3$ | $C_2H_5$ | $i-C_3H_7$ | O | S | |

Die erfindungsgemässen heterocyclischen Phosphorsäureester der Formel I sind geeignet, Schädlinge aus der Klasse der Insekten, Spinnentiere und Nematoden wirksam zu bekämpfen.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Grosser Frostspanner), Bupalus piniarius (Kiefernspanner), Hyphantria cunea (Weisser Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweissling), Aporia crataegi (Baumweissling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüssler), Otiorrhynchus ovatus (Erdbeerwurzelrüssler), Hylobies abietis (Grosser Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüssler), Ceuthorrhynchus napi (Grosser Kohltriebrüssler), Sitophilus granaria (Kornkäfer), Anisandrus dispar (Ungleicher Holzborkenkäfer), Ips typographus (Buchdrucker), Blastophagus pinperda (Gefurchter Wandgärtner);

aus der Ordnung der Zweiflügler (Diptera) beispielsweise Lycoria pectoralis, Mayetiola destructor (Hessenfliege), Dasyneura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus

cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera) beispielsweise Perkinsiella saccharicida (Zukkerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weisse Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysaphis radicola (Mehlige Apfelfalterlaus), Brachycaudus cardui (Grosse Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Grosse Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weisstannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrekke), Schistocerca peregrina (Wanderheuschrekke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Arachnoidea gehören Spinnentiere (Acarina) beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Zur Klasse der Nemathelminthes zählen beispielsweise Wurzelgallennematoden, z.B. Meloidogyne incognita, Meloidogyne hapla, Meloidogyne javanica, Zysten bildende Nematoden, z.B. Heterodera rostochiensis, Heterodera schachtii, Heterodera avenae, Heterodera glycines, Heterodera triflolii, Stock- und Blattälchen, z.B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nusschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in grösseren Bereichen variiert werden. Im allgemeinen liegen sie zwischen 0,0001 und 10%, vorzugsweise zwischen 0,01 und 1%.

Beispiele für Formulierungen sind:

I. 3 Gewichtsteile des Wirkstoffs Nr. 1 werden mit 97 Gewichtsteilen feinteiligem Kaolin innig vermischt. Man erhält auf diese Weise ein Stäubemittel, das 3 Gew.% des Wirkstoffs enthält.

II. 30 Gewichtsteile des Wirkstoffs Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 20 Gewichtsteile des Wirkstoffs Nr. 4 werden in einer Mischung gelöst, die aus 80 Gewichtsteilen Xylol, 10 Gewichtsteilen des Anlagerungsprodukes von 8 bis 10 Mol Äthylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gew.% des Wirkstoffs enthält.

IV. 20 Gewichtsteile des Wirkstoffs Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemässen Mitteln im Gewichtsverhältnis 1:10 bis 10:1 zugemischt werden.

Beispielweise können folgende Mittel zugemischt werden: 1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, o-Chlorphenyl-N-methylcarbamat, 3-Ispropoyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethylbenzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N-[(methylcarbamoyl)-oxy]-thio-acetimidat, Methyl-N′,N′-dimethyl-N-[(methylcarbamoyl)-oxy]-1-thiooxamidat, N-(2-Methyl-chlorphenyl)-N′,N′-dimethylformamidin, Tetrachlorthiophen, 1-(2,6-Difluor-benzoyl)-3-(4-chlor-phenyl)-harnstoff, O,O-Dimethyl-O-(p-nitrophenyl)-phosphorthioat, O,O-Diethyl-O-(p-nitrophenyl)-phosphorthioat, O-Ethyl-O-(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenylphosphonothioat, O,O-Dimethyl-O-(2,4,5-trichlorphenyl)-phosphorthioat, O-Ethyl-O-(2,4,5-trichlorphenyl)-ethyl-phosphonothioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-[p-methylsulfinyl-phenyl]-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[-2-chlor-1-(2,4,5-trichlorphenyl)]-vinyl-phosphat, O,O-Dimethyl-S-(1′-phenyl)-ethylacetat-phos-

phordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methylcarbamoyl-methyl)-phosphorthioat, O,O-Dimethyl-S-(N-methoxyethyl-carbamoyl-methyl)-phosphordithioat, O,O-Dimethyl-S-(N-formyl-N-methyl-carbamoylmethyl-phosphordithioat, O,O-Dimethyl-O-[1-methyl-2-(methylcarbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthio-methyl)-phosphordithioat, O,O-Diethyl-S-[(p-chlor-phenylthio)-methyl]-phosphordithioat, O,O-Dimethyl-S-(2-ethylthio-ethyl)-phosphorthioat, O,O-Dimethyl-S-(2-ethylthio-ethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorthioat, O,O-Diethyl-thiophosphoryliminophenyl-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amido-thioat, O,S-Dimethyl-N-acetyl-phosphoramidothioat, γ-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,trans-2,2-dimethyl-3-(2,2-dichlorvinyl)-cyclopropancarboxylat, α-Cyano-3-phenoxybenzyl(±)-cis,trans-2,2-dimethyl-3-(2,2-dichlorvinyl)-cyclopropancarboxylat, (s)-α-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,trans-chrysanthemat, 2-Methyl-5-(2-propinyl)-3-furylmethyl-chrysanthemat, (α-Cyano-3-phenoxybenzyl)-α-isopropyl-4-chlorphenylacetat.

Die folgenden Beispiele belegen die biologische Wirkung der neuen Verbindungen. Vergleichsmittel sind die bekannten Wirkstoffe O,O-Diethyl-S-(2-chlor-1-phthalimido-ethyl)-phosphordithioat (A; US-PS 2984669) und O,O-Dimethyl-S-(N-methyl-carbamoyl-methyl)-phosphordithioat (B; DE-PS 819998).

Beispiel A

Kontaktwirkung auf Moskito-Larven (Aedes aegypti).

200 ml Leitungswasser werden mit der wässrigen Wirkstoffemulsion versetzt und darauf mit 30 bis 40 Moskito-Larven im 4. Larvenstadium besetzt. Die Versuchstemperatur beträgt 20°C. Nach 24 Stunden wird die Wirkung ermittelt.

In diesem Test zeigen die erfindungsgemässen Wirkstoffe Nr. 1, 2, 3, 5, 14 und 30 eine bessere Wirkung als das Vergleichsmittel B.

Beispiel B

Kontaktwirkung auf Baumwollwanzen (Dysdercus intermedius)

Petrischalen von 10 cm Durchmesser werden mit 1 ml der acetonischen Wirkstofflösung ausgekleidet.

Nach dem Verdunsten des Lösungsmittels besetzt man die Schalen mit je 20 Larven des vorletzten Stadiums und registriert die Wirkung nach 24 Stunden. Die erfindungsgemässen Wirkstoffe Nr. 2, 5, 8, 12, 13, 14, 16, 30, 31 und 33 sind dabei dem Vergleichsmittel A überlegen.

Beispiel C

Frass- und Kontaktwirkung auf Raupen der Kohlschabe (Plutella maculipennis)

Blätter von jungen Kohlpflanzen werden 3 Sekunden lang in die wässrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung, die bei den Wirkstoffen Nr. 1, 2, 3, 5, 6, 10, 11, 13, 14, 15, 18, 20, 21, 24, 30, 31 und 42 sehr gut ist.

Beispiel D

Kontaktwirkung auf Schaben (Blatta orientalis)

Der Boden eines 1 l-Einmachglases wird mit der acetonischen Lösung des Wirkstoffs behandelt. Nach dem Verdunsten des Lösungsmittels setzt man je Glas 5 adulte Schaben. Die Mortalitätsrate wird nach 48 Stunden bestimmt; sie ist bei Verwendung der erfindungsgemässen Wirkstoffe Nr. 1, 5, 13, 14, 30 und 42 höher als bei Verwendung des Verlgeichsmittels A.

Beispiel E

Wirkung auf Spinnmilben (Tetranychus telarius)

Getopfte Buschbohnen, die das erste Folgeblattpaar entwickelt haben und einen starken Besatz aller Stadien der Spinnmilbe Tetranychus telarius tragen, werden in einer Spritzkabine mit der wässrigen Wirkstoffaufbereitung tropfnass gespritzt. Die Pflanzen werden dabei auf einem Drehteller von allen Seiten mit 50 ml Spritzbrühe besprüht. Der Sprühvorgang dauert ca. 22 Sekunden.

Bei der Untersuchung der Pflanzen auf lebende Spinnmilben nach acht Tagen zeigt sich, dass eine wesentlich geringere Menge der erfindungsgemässen Wirkstoffe Nr. 1, 2, 3, 5, 10, 13, 14, 15, 18, 20, 23, 24, 30, 31, 33 und 42 genügt, um die Spinnmilben abzutöten als sie bei Verwendung des Vergleichsmittels B erforderlich ist.

Beispiel F

Kontaktwirkung auf Zecken (Ornithodorus moubata)

Geprüft wird mit Zecken im 3. Larvenstadium. Dazu taucht man die Tiere, die sich in einem Papierbeutel befinden, für 3 Sekunden in die wässrige Wirkstoffaufbereitung.

Die Beutel werden frei aufgehängt. Nach 48 Stunden wird die Wirkung auf die Zecken beurteilt. Bei einer Konzentration der Emulsion von 0,005 bzw. 0,01 Gew.% der Wirkstoffe Nr. 1, 2, 3, 8, 13, 15, 16, 18, 20, 23, 30, 31 und 33 der Formel I werden die Zecken zu einem hohen Prozentsatz getötet.

Beispiel G

Wirkung auf Wurzelgallennematoden (Meloidogyne incognita)

200 g Gartenerde, die stark mit Meloidogyne incognita infiziert ist, werden mit 30 ml der wässrigen Wirkstoffaufbereitung innig vermischt. Die Erde wird darauf in Plastiktöpfe gefüllt und mit Tomaten bepflanzt.

Nach 6 bis 8 Wochen werden die Wurzeln auf Gallenbildung untersucht. Dabei zeigt sich, dass die Gallenbildung durch die Wirkstoffe Nr. 30, 31, 32, 33 verhindert wird, während die Kontrolle zu diesem Zeitpunkt bereits eine starke Vergallung an den Wurzeln zeigt.

**Patentansprüche**

1. Heterocyclische Phosphorsäurederivate der Formel I

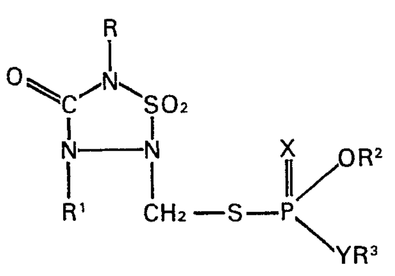

(I),

in der

R gegebenenfalls durch Methoxy oder Halogen substituiertes Alkyl mit bis zu 10 Kohlenstoffatomen oder Cycloalkyl mit 4 bis 8 Kohlenstoffatomen,

$R^1$ Alkyl mit bis zu 6 Kohlenstoffatomen oder Cycloalkyl mit 4 bis 8 Kohlenstoffatomen,

$R^2$ Alkyl mit bis zu 5 Kohlenstoffatomen,

$R^3$ Alkyl mit bis zu 5 Kohlenstoffatomen,

X Sauerstoff oder Schwefel und

Y Sauerstoff, Schwefel oder den bivalenten Rest $-NR^4-$, wobei $R^4$ für Wasserstoff oder Alkyl mit bis zu 5 Kohlenstoffatomen steht, bedeuten.

2. 2-(O-Ethyl-S-isobutyl-phosphorylmercapto-methyl)-3-methyl-5-isopropyl-1,2,3,5-thiatriazolidin-4(2H)-on-1,1-dioxid.

3. 2-(O-Ethyl-S-sec-butyl-phosphoryl-mercapto-methyl)-3-methyl-5-isopropyl-1,2,3,5-thiatriazolidin-4(2H)-on-1,1-dioxid.

4. Verfahren zur Herstellung von heterocyclischen Phosphorsäurederivaten der Formel I gemäss Anspruch 1, dadurch gekennzeichnet, dass man ein 1,2,3,5-Thiatriazolidin-4(2H)-on-1,1-dioxidderivat der Formel II

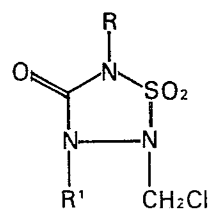

(II),

in der

R und $R^1$ die im Anspruch 1 genannten Bedeutungen haben, mit einem Phosphorsäureestersalz der Formel III

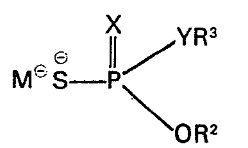

(III),

in der

$R^2$, $R^3$, X und Y die im Anspruch 1 genannten Bedeutungen haben und $M^{\oplus}$ für ein gegebenenfalls substituiertes Ammoniumion, ein Alkalimetallion oder ein Äquivalent Erdalkalimetallion steht, in Gegenwart eines inerten organischen Lösungsmittels umsetzt.

5. Schädlingsbekämpfungsmittel, enthaltend ein heterocyclisches Phosphorsäurederivat der Formel I gemäss Anspruch 1.

6. Verfahren zur Bekämpfung von Schädlingen, dadurch gekennzeichnet, dass man eine wirksame Menge eines heterocyclischen Phosphorsäurederivats der Formel I gemäss Anspruch 1 auf die Schädlinge bzw. deren Lebensraum einwirken lässt.

**Claims**

1. A heterocyclic phosphoric acid derivative of the formula I

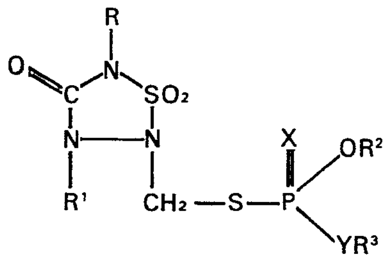

(I),

where R denotes unsubstituted or methoxy- or halogen-substituted alkyl of up to 10 carbon atoms or cycloalkyl of from 4 to 8 carbon atoms, $R^1$ denotes alkyl of up to 6 carbon atoms or cycloalkyl of from 4 to 8 carbon atoms, $R^2$ denotes alkyl of up to 5 carbon atoms, $R^3$ denotes alkyl of up to 5 carbon atoms, X denotes oxygen or sulfur, and Y denotes oxygen, sulfur or the bivalent radical $-NR^4-$, $R^4$ denoting hydrogen or alkyl of up to 5 carbon atoms.

2. 2-(O-Ethyl-S-isobutyl-phosphorylmercapto-methyl)-3-methyl-5-isopropyl-1,2,3,5-thiatriazolidin-4(2H)-one-1,1-dioxide.

3. 2-(O-Ethyl-S-sec-butyl-phosphorylmercapto-methyl)-3-methyl-5-isopropyl-1,2,3,5-thiatriazolidin-4(2H)-one-1,1-dioxide.

4. A process for the preparation of a heterocyclic phosphoric acid derivative of the formula I as claimed in claim 1, wherein a 1,2,3,5-thiatriazolidin-4(2H)-one-1,1-dioxide derivative of the formula II

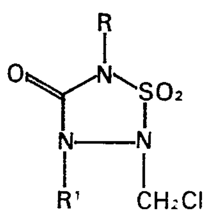

(II),

where R and $R^1$ have the meanings given in claim 1, is reacted with a phosphoric acid ester salt of the formula III

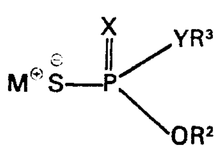

(III),

where $R^2$, $R^3$, X and Y have the meanings given in claim 1 and $M^\oplus$ denotes an unsubstituted or substituted ammonium ion, an alkali metal ion or one equivalent of an alkaline earth metal ion, in the presence of an inert organic solvent.

5. A pesticide containing a heterocyclic phosphoric acid derivative of the formula I as claimed in claim 1.

6. A process for combating pests, wherein an effective amount of a heterocyclic phosphoric acid derivative of the formula I as claimed in claim 1 is allowed to act on the pests or their habitat.

**Revendications**

1. Dérivés d'acide phosphorique hétérocycliques de formule I

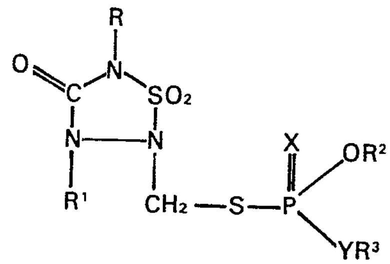

(I),

dans laquelle
R représente un alkyle ayant jusqu'à 10 atomes de carbone,
éventuellement substitué par méthoxy ou halogène, ou un cycloalkyle à 4 à 8 atomes de carbone
$R^1$ un alkyle ayant jusqu'à 6 atomes de carbone ou un cycloalkyle ayant 4 à 8 atomes de carbone
$R^2$ un alkyle ayant jusqu'à 5 atomes de carbone
$R^3$ un alkyle ayant jusqu'à 5 atomes de carbone
X ogygène ou soufre et
Y oxygène, soufre ou le reste bivalent $-NR^4-$, $R^4$ étant l'hydrogène ou un alkyle ayant jusqu'à 5 atomes de carbone.

2. 2-(O-éthyl-S-isobutyl-phosphoryl-mercapto-méthyl)-3-méthyl-5-isopropyl-1,2,3,5-thiatriazolidin-4(2H)-on-1,1-dioxyde.

3. 2-(O-éthyl-S-sec-butyl-phosphoryl-mercapto-méthyl)-3-méthyl-5-isopropyl-1,2,3,5-thiatriazolidin-4(2H)-on-1,1-dioxyde.

4. Procédé de préparation de dérivés d'acide phosphorique hétérocycliques de formule I selon la revendication 1, caractérisé par le fait qu'on fait réagir, en présence d'un solvant organique inerte, un dérivé de 1,2,3,5-thiatriazolidin-4(4H)-on-1,1-dioxyde de formule II

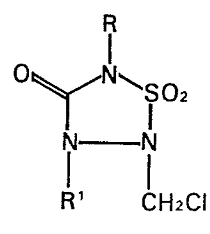

(II),

dans laquelle
R et $R^1$ ont les significations indiquées dans la revendication 1, avec un sel d'ester d'acide phosphorique de formule III

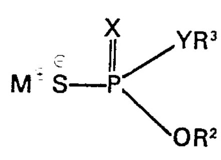

(III),

dans laquelle
$R^2$, $R^3$, X et Y ont les significations indiquées dans la revendication 1 et $M^\oplus$ est un ion ammonium

éventuellement substitué, un ion métal alcalin ou un ion métal alcalino-terreux équivalent.

5. Agent de lutte contre les parasites, contenant un dérivé d'acide phosphorique hétérocyclique de formule I selon la revendication 1.

6. Procédé de lutte contre les parasites, caractérisé par le fait qu'on fait agir sur les parasites ou leur biotope une quantité efficace d'un dérivé d'acide phosphorique hétérocyclique de formule I selon la revendication 1.